# EUROPÄISCHE PATENTSCHRIFT

(11) **EP 1 645 241 B1**
(45) Veröffentlichungstag und Bekanntmachung des Hinweises auf die Patenterteilung: **28.12.2011**
(21) Anmeldenummer: 04023718.2
(22) Anmeldetag: 05.10.2004
(51) Int. Cl.: A61B 19/00, G01S 7/481, G02B 6/00

(54) **Positionsmarkersystem mit Punktlichtquellen**
Position marker system with point light sources
Système de marque de position avec des sources lumineuses ponctuelles

(43) Veröffentlichungstag der Anmeldung: 12.04.2006
(73) Patentinhaber: BrainLAB AG, 85622 Feldkirchen (DE)
(72) Erfinder: Goldbach, Günter, 85661 Forstinning (DE)
(74) Vertreter: Schwabe - Sandmair - Marx

(56) Entgegenhaltungen:
- WO-A-97/47240
- WO-A-03/038481
- US-A- 5 928 140
- US-A1- 2003 099 435
- US-A1- 2003 174 401
- US-A1- 2003 181 800

## Beschreibung

Die Erfindung betrifft ein Positionsmarker-Trackingsystem mit einem optischen, insbesondere medizinischen Trackingsystem und mindestens einem lichtabstrahlenden oder lichtreflektierenden Marker, der positionell von dem Trackingsystem erfassbar ist.

Positionsmarkersysteme für optische, insbesondere medizinische Trackingsysteme sind grundsätzlich bekannt. Aus der DE 19 639 615 C2 (US 6,351,659 B1) ist beispielsweise ein Reflektorenreferenzierungssystem bekannt, bei dem die Positionsmarker als "passive" Marker ausgebildet sind und insbesondere Kugeln mit einem reflektierenden Überzug aufweisen. Die Reflektionen dieser Kugeln werden von einem kameragestützten Trackingsystem erfasst, die räumliche Position wird bestimmt und Instrumente, Patienten oder Behandlungsgeräte können so räumlich erfasst und verfolgt werden, um einem Chirurgen eine visuelle Unterstützung bei seiner Arbeit geben zu können. Ferner sind in ähnlicher Weise funktionierende "aktive" Marker bekannt, die aus Stromquellen wie Batterien oder Akkus versorgt werden und selbst Licht abstrahlen, wobei ein solches System beispielsweise aus der US-A-5,197,476 bekannt ist. Auch die US-A-5,383,454 und die US 5,831,735 zeigen Beispiele für kontaktlose optische Ortung mit Hilfe von Positionsmarkern.

Nachteilig bei bisher bekannten aktiv abstrahlenden Markern, wie z.B. LED-Markern, aber auch bei Marker-Reflektoren ist die relativ große Abmessung der Marker bzw. des effektiven abstrahlenden Teils der Marker. Die Marker nehmen relativ viel Raum ein und sind an kleineren oder sehr dünnen Objekten schwer anzubringen. LEDs liefern außerdem ein recht inhomogenes Lichtmuster. Was die passiven Marker angeht, so ergibt sich das Problem, dass sie schon relativ groß ausgestaltet werden müssen, um überhaupt von den Kameras erfasst werden zu können. Relativ große Marker haben aber den Nachteil, dass sie während ihrer Verwendung teilweise abgedeckt werden können, verschmutzt werden können oder keine ganz regelmäßige Form aufgrund von Herstellungstoleranzen aufweisen. Bei solchen passiven Markern wird deren Zentrum, das den Markerort exakt wiedergibt, durch eine Berechnung des "Schwerpunktes" ermittelt, indem die abgebildete äußere Form analysiert und der Mittelpunkt berechnet wird. Bei teilweise abgedeckten, verschmutzen oder unterschiedlich großen Markern ist eine genaue Berechnung des Mittelpunktes aber nicht immer möglich. Es entstehen Fehler, die bei Anwendungen, welche eine hohe Genauigkeit fordern, fatal sein können, insbesondere bei chirurgischen Anwendungen. Außerdem ist nicht immer gewährleistet, dass ein Marker, der von zwei Kameras aufgenommen wird, aus beiden Blickwinkeln her dieselbe Form aufweist, beispielsweise wenn eine Verschmutzung oder Abdeckung vorliegt. Gleiches gilt für die "aktiven" LEDs wegen der inhomogenen Abstrahlung.

Aus der WO 97/47240 ist ein Marker-Positionserfassungssystem bekannt, bei dem Glasfasern als Lichtleiter verwendet werden, die an einem Ende, an den Markern, Licht abstrahlen.

Die Erfindung hat es sich zur Aufgabe gemacht, die vorgenannten Probleme zu lösen. Insbesondere soll ein System bereitgestellt werden, welches eine exakte Positionsbestimmung zulässt.

Diese Aufgabe wird erfindungsgemäß durch ein System gemäß dem Anspruch 1 gelöst.

Der Begriff "Punktlichtquelle" umfasst Lichtquellen mit sehr kleinen Abmessungen, deren Position genau auf einem Kamerasensor bestimmt werden kann und deren Helligkeit ausreicht, um den Sensor zu belichten.

Der oben genannte effektive abstrahlende bzw. reflektierende Teil des oder der Marker wird im Weiteren auch als "Punktlicht" bezeichnet: Diese Punktlichter sind insbesondere solche, die in Abstrahlrichtung ein homogenes Licht abstrahlen, also aus allen Richtungen ihres Leuchtbereiches dasselbe Bild abgeben.

Eine Definition der Größe der Punktlichter, also der Größe des effektiv abstrahlenden bzw. reflektierenden Teil des oder der Marker, ist eine Definition über ihre Positionierungstoleranz (Positionierungsgenauigkeit). Eine Positionierungstoleranz bzw. eine Positionierungsgenauigkeit ist auf dem Gebiet der optischen Trackingsysteme allgemein die Genauigkeit der Lagebestimmung von Markern im Raum (meist relativ zueinander, aber auch absolut). Die Punktlichter haben eine Größe, die geringer ist als das 25-fache ihrer Positionierungstoleranz.

Die Punktlichter haben eine Größe von einem Bruchteil von 1 mm. Ferner können sie eine Größe von mindestens 30 µm haben, insbesondere mindestens 50 µm und vorzugsweise mindestens 62,5 µm.

Ein erfindungsgemäßes System ist vorzugsweise so ausgestaltet, dass das Punktlicht oder die Punktlichter so bearbeitet oder ausgebildet ist bzw. sind, dass sie Licht in räumlich gleichmäßiger Verteilung abstrahlen. Das Ende eines lichtleitenden Innenteils einer Lichtleiterfaser ist so poliert oder geschliffen, dass es eine konvexe Streulinse bildet.

Gemäß einer bevorzugten Ausführungsform des erfindungsgemäßen Systems weist dieses mehrere Marker auf, welche die Enden von Lichtleitern sind, die aus einer gemeinsamen Lichtquelle gespeist werden, wobei die Lichtquelle eine aktiv abstrahlende Quelle oder eine Lichtsammeleinrichtung sein kann, die Umgebungslicht sammelt.

Die Erfindung wird im Weiteren anhand von Ausführungsformen näher erläutert. Sie kann alle hierin beschriebenen Merkmale einzeln oder in jedweder Kombination aufweisen. In den beiliegenden Zeichnungen zeigen:
- Figur 1: einen Vergleich der Größe herkömmlicher Reflektorenmarker mit erfindungsgemäßen Punktlicht-Markern;
- Figur 2: eine Ausführungsform einer Lichtleiteranordnung mit Lichtsammeleinrichtung;
- Figur 3: das Ende eines Lichtleiters mit einer Punktlichtquelle;
- Figur 4 und Figur 5: Beispiele für Bilder mit und ohne Stern- bzw. Kreuzeffekte;
- Figur 6: Beispiele für die Bestimmung von Bildpunkten auf Kamerasensoren mit Hilfe von Stern- bzw. Kreuzeffekten;
- Figur 7: eine Gegenüberstellung der Bildpunkt-Positionsermittlung in herkömmlicher Weise und mit Lichtstreueffekten; und
- Figur 8: eine schematische Darstellung eines optischen Trackingsystems.

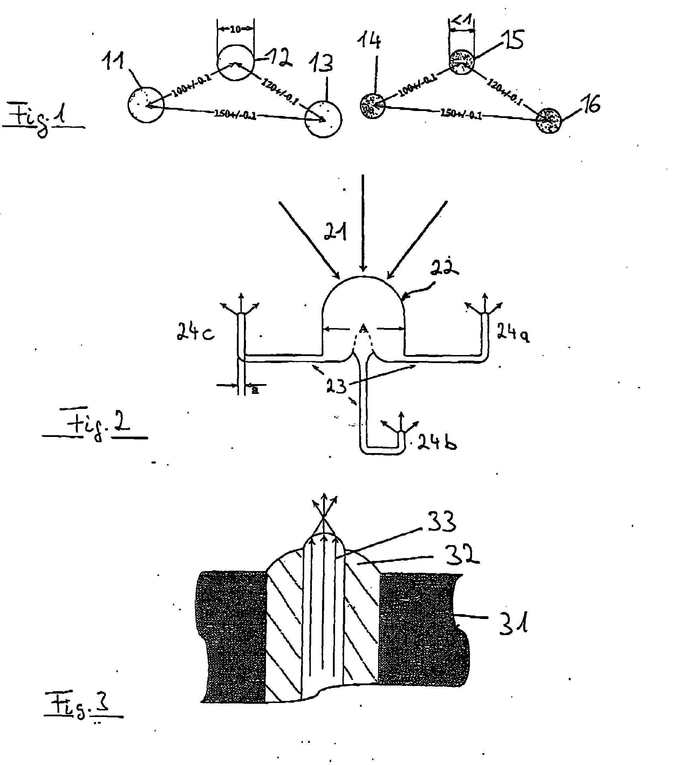

Die Figur 1 zeigt eine Gegenüberstellung eines herkömmlichen (links) und eines erfindungsgemäßen Positionsmarkersystems (rechts). Bei den dargestellten Markern handelt es sich um die tatsächlich reflektierende Fläche eines Reflektorenmarker 11, 12, 13. In der Regel werden heute kugel- oder scheibenförmige retroreflektiernede Oberflächen verwendet, die über einen relativ großen Bereich Licht in die Ausgangsrichtung zurückwerfen, um ein möglichst großes Abbild des Markers im Trackingsystem zu erzeugen. Drei solcher Marker können beispielsweise an einem Patienten-Körperteil oder auf einem festen Körper (Markergeometrie oder englisch rigid body) angebracht werden, um dessen Position im Raum mittels eines Trackingsystems zu ermitteln. Die Marker 11, 12 und 13 des herkömmlichen Markersystems weisen untereinander Abstände von 100, 120 bzw. 150 mm auf, und sie haben einen Durchmesser von 10 mm. Die Positionierungstoleranz bzw. die Positionierungsgenauigkeit, also die Genauigkeit der Lagebestimmung von einzelnen Markern im Raum (relativ zueinander, aber auch absolut) ist hier mit +/- 0,1 mm angegeben. Dies ist eine übliche Positionierungstoleranz.

Die Marker 14, 15, 16 des in Figur 1 rechts dargestellten, erfindungsgemäßen Systems sind sehr viel kleiner ausgestaltet, wobei die Fläche der erfindungsgemäßen Marker hier nochmals wesentlich kleiner sein kann oder sogar soll. Retroreflektierende Oberflächen zur Vergrößerung der effektiven Markerfläche werden deshalb nicht benötigt. Im vorliegenden Fall haben die Marker bei gleicher Positionierungstoleranz und gleichen Abständen zueinander nur einen Durchmesser von einem Bruchteil von 1 mm.

Die Verwendung von Punktlichtquellen-Markern führt zu präziseren zweidimensionalen Positionsmessungen (mit einer Kamera), erhöht aber auch die Genauigkeit bei einer dreidimensionalen Erfassung, wenn ein stereoskopisches Kamerasystem verwendet wird. Dies liegt einerseits daran, dass ein Punktlichtquellen-Marker mit größerer Wahrscheinlichkeit aus zwei Richtungen dasselbe Bild abgibt als ein relativ großer Marker, da mechanische Deformationen, Abdeckung sowie eine ungleichmäßige Helligkeitsverteilung mit einfachen Mitteln eliminiert werden können und die Positionsinterpolation nicht länger beeinflussen. Die als einzelne leuchtende Objekte ausgestalteten Punktlichtquellen-Marker haben eine Reihe von Vorteilen. Der potentielle Fehler bei der Berechnung oder Abschätzung des Mittelpunktes (Schwerpunktes) ist prinzipiell kleiner. Das Risiko einer teilweisen Verdeckung von Markern und deshalb einer nicht korrekten Abschätzung des Schwerpunktes ist begrenzt, weil die Marker entweder ganz abgedeckt werden oder überhaupt nicht. Wenn ein Marker ganz abgedeckt wird, muss dies nicht zu einem Ausfall des Systems führen, da grundsätzlich mehr als die benötigte Marker-Mindestanzahl zum Orten eines einzigen Objektes bereitgestellt werden können und weil nach einer gewissen Zeit ein Marker nicht mehr abgedeckt sein wird, so dass die Ortung korrekt weitergehen kann. Bei medizinischen Anwendungen, beispielsweise beim Tracking von chirurgischen Instrumenten wird die zu erwartende Größe von abdeckenden Objekten (Blut, Wassertropfen, Finger eines Chirurgen, etc.) typischerweise größer sein als die des Punktlicht-Markers gemäß der vorliegenden Erfindung. Dadurch ist sichergestellt, dass nicht ein nur teilweise sichtbarer Marker positionell falsch detektiert wird, was die Positionsbestimmung insgesamt verfälschen würde. Weitere Vorteile betreffen das geringere Gewicht von Instrumenten, die mit solchermaßen kleinen Markern versehen sind und die Möglichkeit, Marker in sehr dünne Instrumente integrieren zu können, beispielsweise in Katheter, Bohrwerkzeuge, etc.

Das Positionsmarkersystem gemäß der vorliegenden Erfindung ist ein aktives Markersystem, hat also Punktlichtquellen , die selbst Licht abstrahlen. Ferner können aktive Systeme, also mit selbst abstrahlenden Markern, im Rahmen der Erfindung unterschiedliche Lichtversorgungen aufweisen. Es können Lichtquellen bereitgestellt werden, die ihre eigene Energieversorgung aufweisen, also beispielsweise eine Lichtquelle, die von einer Batterie, einem Akku, einer Fuel Cell oder einer Solarzelle gespeist wird, die oder der in einem kabellosen Instrument vorgesehen wird, welches mittels der Punktlichtquellen geortet werden soll. Natürlich kann auch von außen ein Lichtleiterkabel an die Positionsmarker-Lichtversorgung angeschlossen werden oder eine externe Energieversorgung für eine Lichtquelle in dem Objekt, das mit dem Markern versehen wird.

Eine besonders vorteilhafte Ausführungsform ist eine solche, bei der ein kabelloses Objekt, also beispielsweise ein chirurgisches Instrument, bereitgestellt wird, das ohne eigene oder äußere (elektrische) Energieversorgung arbeiten kann und trotzdem eine sehr hohe Energiedichte der Positionsmarker-Punktlichter aufweist. Ein Positionsmarkersystem für ein solches chirurgisches Instrument (oder für chirurgische Behandlungsgeräte bzw. zur Markierung von Patienten-Körperteilen) weist eine Lichtsammeleinrichtung auf, von der sich mindestens ein Lichtleiter erstreckt, dessen Ende eine Punktlichtquelle bildet. In Figur 2 ist schematisch eine solche Anordnung gezeigt. Außenlicht 21, nämlich Umgebungslicht oder Licht aus einer aktiven Lichtquelle (ständig leuchtende oder intermittierende Lichtquelle) wird in einer relativ großen Kollimatorlinse 22 gesammelt. Im Falle medizinischer Anwendungen kann diese Lichtsammellinse 22 beispielsweise am hinteren Ende eines Instruments positioniert sein. Sie kann gut durch sehr helle Operationslichter mit sichtbarem Licht versorgt werden oder durch eine separate Lichtquelle die unsichtbares Licht bzw. unsichtbare elektromagnetische Strahlung (ultraviolett oder infrarot) emittiert. Die Kollimatorlinse 22 ist so aufgebaut, dass sie sich in eine Anzahl kleiner Lichtleiter 23 aufspaltet, die das von der Linse 22 gesammelte Licht zu den präzise positionierten Punktlichtquellen 24a bis c transportieren. Die Lichtleiter 23 können aus einem flexiblen oder steifen Kunststoffmaterial oder Glasmaterial hergestellt sein, mit optimierten Lichttransmissionseigenschaften, wie dies aus optischen Glasfaser-Datenübertragungsleitungen bekannt ist. Die Helligkeit des an den Enden bzw. Punktlichtquellen 24a bis c abgestrahlten Lichtes ist eine Funktion der Größe (Maß a) der Kollimatorlinse 22 in Bezug auf die Apertur der Punktlichtquelle 24a bis c (Maß a), die Anzahl der verwendeten Lichtleiter und in Bezug auf die optischen Eigenschaften des verwendeten Materials.

Basierend auf diesem Prinzip kann ein relativ heller Punktlichtmarker mit passiver Beleuchtung erzielt werden. Je kleiner die Apertur (a) ist, desto höher ist die Lichtdichte an den Enden 24a bis 24c. Stärkeres Umgebungslicht resultiert in einer höheren Marker-Helligkeit und stellt deshalb ein gutes Signal/Rauschen-Verhältnis über einen weiten Bereich der Eigenschaften des einfallenden Lichtes zur Verfügung.

Wie oben bereits angedeutet, besteht eine Möglichkeit zur Herstellung von aktiven Punktlichtmarkern mit sehr geringer Größe darin, herkömmlich erhältliche optische Faserkabel entweder aus Glas oder Kunststoff zu verwenden, wie sie beispielsweise in der Telekommunikation genutzt werden. Der lichtleitende innere Teil einer Glasfaseranordnung hat typischerweise eine Größe von 50 bis 62,5 µm für MultiMode-Fasern. Optische Kunststofffasern sind mit Durchmessern bis zu einigen Millimetern erhältlich. Es ist bekannt, wie man aus einem Laser oder einer LED-Lichtquelle intensives Licht in eine solche Faser einbringt.

Die Figur 3 zeigt eine Möglichkeit der Ausgestaltung einer Punktlichtquelle mit einem Lichtleiter. Der Lichtleiter 33 selbst ist üblicherweise mit einer Umhüllung 32 umgeben, die wiederum in dem Objekt verankert ist, das mit der Punktlichtquelle versehen werden soll, beispielsweise in der Wandung 31 eines chirurgischen Instruments. Der Lichtleiter 33 selbst tritt an seinem Ende etwas aus seiner Umhüllung 32 hervor. Dieses Ende ist bei der dargestellten Ausführungsform poliert oder geschliffen und bildet somit eine konvexe Verteilerlinse, die das Licht gleichmäßig in alle Abstrahlrichtungen abgibt.

Die Erfindung kann vorteilhaft mit einer Bilderzeugungsvorrichtung für ein optisches, insbesondere medizinisches Trackingsystem, mit dem die Position eines aufgenommenen Bildpunktes bestimmt wird, verwendet oder kombiniert werden.

Bekannte optische Trackingsysteme und deren Bilderzeugungsvorrichtungen umfassen meist ein Kamerapaar, welches einzelne Bildpunkte, meist Positionsmarker oder natürliche Landmarken, aufnimmt und aus dem stereoskopischen Bild Informationen über die Position, nämlich die räumliche Position der Bildpunkte, erfasst. Dabei gibt es Systeme, die im Kameraträger schon eine Bildverarbeitungseinheit aufweisen und als Ausgangssignal schon die räumlichen Koordinaten der erfassten Bildpunkte liefern. Grundsätzlich kann die Verarbeitung der Bilder aber auch an externer Stelle erfolgen.

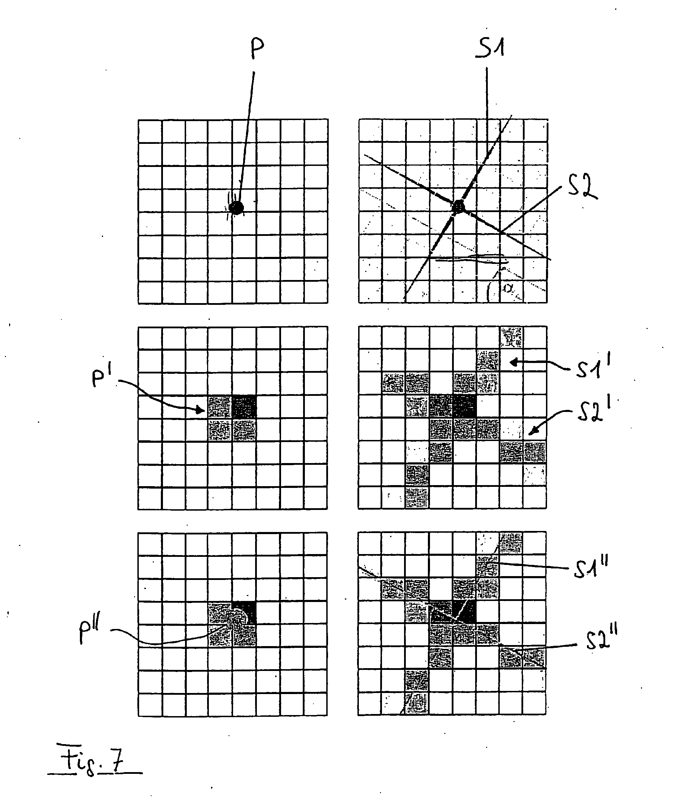

Insofern sie Kamerasysteme mit einer vorgegebenen Bildauflösung verwenden, haben alle herkömmlichen Systeme den Nachteil, dass ihre Positionsbestimmungs-Genauigkeit wesentlich von der Auflösung der Bilderzeugungsvorrichtungen abhängig ist. Wenn relativ kleine Marker oder Landmarken aufgenommen und in ihrer Position bestimmt werden sollen oder bei Markern und Landmarken, die relativ weit von der Bilderzeugungsvorrichtung entfernt sind, stößt die Genauigkeit herkömmlicher Bilderzeugungsvorrichtungen schnell an ihre Grenzen, und ein Beispiel hierfür ist in der beiliegenden Figur 7 in der linken Bildreihe von oben nach unten erkennbar. Hier wird dargestellt, wie ein Bildpunkt P auf einem 8x8―Sensor einer herkömmlichen Bilderzeugungs-vorrichtung in der Position erfasst würde. Es ist zu bemerken, dass die Größe der Bildpunkte (z.B. eines Markers) auf dem Sensor im Sinne der Erfindung sehr viel kleiner sein kann als die Pixelgröße des Sensors, was in diesem herkömmlichen Fall nur zu einer maximalen Positionsauflösung von einem Pixel führen kann.

In den drei linken Bildern der Figur 7 ist noch ein relativ guter Fall dargestellt, nämlich einer wo der Bildpunkt P vier Pixel des Sensors übergreift und damit in seiner Abbildung P' (mittleres Bild auf der linken Seite in Figur 7) 4 Pixel in unterschiedlicher Stärke belichtet. Eine Interpolation der "Helligkeit" (eigentlich der Entwicklungs- oder Ausleuchtungsstärke) der Pixelgruppe führt zu einer Sub-Pixel-Auflösung, welche den "Schwerpunkt" der Abbildung P' in etwa im Bereich des tatsächlichen Bildpunktes P ermittelt. Rechnerisch wird also die Position des Punktes P annähernd dort bestimmt, wo er als P" im unteren Bild der linken Seite in Figur 7 zu sehen ist.

Wie oben schon angemerkt, ist eine solche Sub-Pixel-Auflösung nur dann möglich, wenn der Bildpunkt P tatsächlich mehr als nur einen Pixel belichtet. Im schlechtesten Fall, wo der Bildpunkt P innerhalb eines einzigen Pixels liegt, kann seine Position nur als innerhalb des Pixels liegend festgestellt werden; bei kleinen Markern oder Bildpunkten kann hierbei ein großer Fehler entstehen.

Die Genauigkeit wird also durch die Auflösung des Sensors und die Dynamik und Linearität der Sensorpixel (und im 3D-Fall durch die Homogenität des Markers) bestimmt. Im Falle von medizinischen Trackingsystemen oder anderen Trackingsystemen, wo es in vielen Fällen auf Genauigkeiten von Bruchteilen von Millimetern ankommt, kann in der Regel ein Genauigkeitsfehler von einer Pixelgröße nicht toleriert werden, weshalb stets versucht wird, Marker möglichst groß zu machen, um die Position über die Mittelung von mehren belichteten Pixeln zu bestimmen..

Anders betrachtet werden die Größe von Markern und ihr maximaler Abstand zur Bilderzeugungsvorrichtung klar durch die Auflösung und den Reproduktions-Maßstab der Optiken bestimmt. Zwar könnte die Größe von Bildpunkten, wenn Marker verwendet werden, dadurch erhöht werden, dass man einfach größere Marker nimmt, was jedoch erhebliche Nachteile in der Handhabbarkeit, bei den Kosten und beim Gewicht der Marker mit sich zieht.

Ferner müssen für solche Bildpunkt-Positionsbestimmungen noch einige Voraussetzungen erfüllt werden, nämlich:
- die Belichtung der Pixel wird nur durch ein einziges Objekt ausgelöst, weil sonst die Gewichtung der Graustufen zur Subpixelauflösung fehlschlägt (diese Annahme ist kritisch);
- der Schwerpunkt ist das interpolierte Zentrum für die Helligkeit (dies ist nicht der Fall, wenn ein Marker teilweise verdeckt, nicht rund oder verschmutzt ist, oder die reflektierende Fläche nicht gleichmäßig hell abgebildet wird);
- ein runder Marker produziert dasselbe Bild auf zwei Sensoren, wenn man von zwei verschiedenen Winkeln auf ihn sieht (dies ist ebenfalls dann nicht der Fall, wenn der Marker nicht rund, verschmutzt oder teilweise verdeckt ist, oder die Helligkeit der reflektierenden Fläche nicht homogen ist.);
- das Bild des Markers ist groß genug, um mindestens einen Pixel zu überdecken (in der Praxis mindestens vier); und
- der Marker ist hell genug, um den Marker von dem Hintergrund-Rauschen unterscheiden zu können.

Alle diese Annahmen und Einschränkungen bringen Nachteile für die herkömmlichen Trackingsysteme mit sich. Man hat versucht, die Probleme dadurch zu lösen, dass Bilderzeugungsvorrichtungen mit immer höheren Auflösungen verwendet werden. Jedoch sind sehr hoch auflösende Bildsensoren auch sehr teuer und steigern damit die Kosten erheblich. Auch können einige der oben genannten Probleme nicht einfach dadurch gelöst werden, dass äußerst hoch auflösende Bilderzeugungsvorrichtungen verwendet werden, beispielsweise wenn Bildpunkte nicht vollständig sichtbar sind, z.B. bei verschmutzen Markern. Ein weiterer Nachteil ist, dass hochauflösendere Sensoren in der Regel eine deutlich kleinere Pixelfläche haben und damit unempfindlicher sind, was zu potentiell längeren Belichtungszeiten führt und damit bei bewegten Objekten kritisch ist.

Die Entwickler von Trackingsystemen befinden sich also in einer Art "Zwickmühle": Einerseits kann man die Marker nicht beliebig groß machen und andererseits kann man die Auflösung nicht beliebig erhöhen.

Dieses Problem lässt sich durch eine Bilderzeugungsvorrichtung für ein optisches, insbesondere medizinisches Trackingsystem, mit dem die Position eines aufgenommenen Bildpunktes bestimmt wird, lösen, die eine Lichtstreueffekt-Erzeugungseinrichtung aufweist. Man geht damit einen völlig neuen Weg, der aus dem oben erwähnten Dilemma herausführt. Anstatt die Marker größer oder die Auflösung höher zu machen, wird die Lichtstreueffekt-Erzeugungseinrichtung verwendet, die das Bild eigentlich "unschärfer" macht. Mit der gerichteten, definierten Streuung des Lichtes eines Markers bietet sich die Möglichkeit, auf einem Sensor der Bilderzeugungsvorrichtung eine größere Anzahl von Pixeln zu belichten, wodurch eine räumlich größere Menge an Information auf dem Bildsensor zur Auswertung zur Verfügung steht. Vorteilhaft wirkt sich dabei aus, dass bei der Erzeugung von Lichtstreueffekten Helligkeitsgradienten entstehen, d.h., an der Stelle, wo der Bildpunkt tatsächlich liegt, wird er am hellsten abgebildet, und mit der Entfernung verringert sich diese Helligkeit. Dadurch steht ein Gradient zur Verfügung, der bei der Ermittlung der genauen Bildpunktposition verwendet werden kann.
Kurioserweise wird also die Bildpunktbestimmung genauer, obwohl durch den Lichtstreueffekt das gesamte Bild eigentlich unschärfer gemacht wird.

Mit einer solchen Bilderzeugungsvorrichtung kann deshalb ein Bildaufnahmesystem verwendet werden, das eine relativ geringe Auflösung hat. Die Marker müssen nicht mehr groß sein, sie können vorteilhafterweise sogar sehr klein sein, solange sie ausreichend Helligkeit abstrahlen oder reflektieren und damit determinierbare Bildpunkte und Streuungsmuster entstehen lassen. Man gibt also die Anforderungen an die Schärfe des Bildes auf, indem man die Detektion der Bildpunkte leichter macht. Die Information der tatsächlichen Bildpunktposition wird durch eine optimale Kombination von Bildauflösung und Dynamik (Empfindlichkeit) des Sensors und der Qualität des Streumusters erzielt. Falls bekannte und spezielle Lichtstreueffekte verwendet werden, wird die Gesamtenergie, die von dem Bildpunkt detektiert wird, über eine Anzahl von Pixeln in einem bekannten Muster und in bekannten Winkeln verteilt.

Ein Beispiel für die oben zuletzt genannte Verteilung wird in einer bevorzugten Ausführungsform realisiert, bei der die Lichtstreueffekt-Erzeugungseinrichtung eine Sterneffekt- bzw. Kreuzeffekt-Erzeugungseinrichtung ist, die Stern- bzw.

Kreuzeffekte in zwei Richtungen erzeugt, die in einem vorbestimmten Winkel zueinander stehen, insbesondere im Winkel von 90°.

Es können auch Stern- bzw. Kreuzeffekte höherer Ordnung erzeugt werden, beispielsweise in mindestens drei Richtungen, die in einem vorbestimmten Winkel zueinander stehen, insbesondere in einem Winkel von 180° geteilt durch die Anzahl der Richtungen, also mit gleichem Winkelabständen.

Mit den obigen Ausführungsformen entsteht der besondere Vorteil entgegen einem gezielten, diffusen Defokusieren, dass man durch die Ausgestaltung der Lichtstreueffekt-Erzeugungseinrichtung schon diejenigen Winkel vorgeben kann, in welchem das Kreuz- bzw. Sternmuster erzeugt werden wird. Mit solchen vorbekannten Richtungen lassen sich die Kreuzungspunkte und damit die Bildpunkte selbst sehr viel einfacher erfassen und ermitteln, da man lediglich noch die parallel zu den Sternstrahlen hellste Anordnung ermitteln muss, die dann eine Linie des "Fadenkreuzes" bildet. Am Kreuzungspunkt befindet sich dann die Abbildung des Bildpunktes, beispielsweise des Markers.

Es soll hier bemerkt werden, dass Sterneffekt- bzw. Kreuzeffekt-Erzeugungseinrichtungen nicht die einzigen vorstellbaren Lichtstreueffekt-Erzeugungseinrichtung sind. Ebenfalls wäre es beispielsweise denkbar, verschiedenste Arten von Weichzeichnereffekten zu verwenden, um die Helligkeit von Bildpunkten beispielsweise gleichmäßig und homogen nach außen zu streuen, wobei sie graduell abnimmt.

Die Lichtstreueffekt-Erzeugungseinrichtung kann verschiedene Ausführungsformen annehmen. Eine Hardware-Konfiguration wäre beispielsweise ein Lichtstreueffekt-Filter, insbesondere ein Sterneffekt- bzw. Kreuzeffektfilter, der vor, hinter, auf oder in einem Kameralinsensystem angeordnet ist.

Ferner kann ein optisches, insbesondere medizinisches Trackingsystem mit einer Bilderzeugungsvorrichtung bereitgestellt werden, wie sie oben beschrieben wurde. Ein solches Trackingsystem kann gemäß einer Ausführungsform eine Bildverarbeitungseinheit umfassen, die anhand des Lichtstreumusters, insbesondere anhand der Sterneffekt-Linien und/oder deren Kreuzungspunkten die Position eines Bildpunktes ermitteln.

Gemäß einer weiteren Ausführungsform umfasst ein solches Trackingsystem eine Bilderzeugungsvorrichtung mit mindestens zwei beabstandeten Kameras, sowie mit einer Bildverarbeitungseinheit, die anhand des Lichtstreumusters, insbesondere der Sterneffekt-Linien und/oder deren Kreuzungspunkten die zweidimensionale Position eines Bildpunktes im Bild jeder Kamera ermittelt und aus den ermittelten zweidimensionalen Positionen eine räumliche Position des Bildpunktes errechnet.

Ferner wird noch ein Verfahren zur Bestimmung der Position eines aufgenommenen Bildpunktes mittels eines optischen, insbesondere medizinischen Trackingsystems, offenbart, bei dem
- mit einer Bilderzeugungsvorrichtung ein Bild erzeugt wird,
- auf dem Bild für vorbestimmte Bildpunkte ein Lichtstreueffekt, insbesondere ein Sterneffekt- bzw. Kreuzeffekt erzeugt wird; und bei dem
- anhand des auf dem Bild abgebildeten Lichtstreumusters, insbesondere anhand der Sterneffekt- bzw. Kreuzeffekt-Linien und/oder deren Kreuzungspunkten die Position eines Bildpunktes ermittelt wird.

Hierbei besteht die Möglichkeit, die Position des Bildpunktes unter Berücksichtigung der vorbekannten und vorbestimmten Winkel von Sterneffekt- bzw. Kreuzeffektlinien zueinander zu bestimmen. Die Stern- bzw. Kreuzeffekte können in zwei Richtungen erzeugt werden, die in einem vorbestimmten Winkel zueinander stehen, insbesondere im Winkel von 90°. Es besteht aber auch die Möglichkeit, die Effekte in mindestens drei Richtungen zu erzeugen, die in einem vorbestimmten Winkel zueinander stehen, insbesondere in einem Winkel von 180° geteilt durch die Anzahl der Richtungen.

In besonders vorteilhafter Ausführungsform werden die Stern- bzw. Kreuzeffekte in mindestens einer Anzahl von Richtungen erzeugt, welche die Anzahl der positionsmäßig zu ermittelnden Bildpunkte um 1 übersteigt. Mit dieser Konfiguration lässt sich ein Bildpunkt immer dort sicher ermitteln, wo sich die höchste Anzahl von Effektlinien kreuzen

Die Position des Bildpunktes kann zusätzlich anhand des Helligkeits-Gradienten des Lichtstreumusters, insbesondere der Stern- bzw. Kreuzeffektlinien ermittelt werden, da dort wo der Bildpunkt abgebildet wird die größte abgebildete Helligkeit sein wird.

Eine besondere Anwendung liegt auf dem Gebiet der bildgeführten, navigationsgestützten medizinischen Behandlung, wo die Position eines Patienten oder eines medizinischen Instruments bzw. einer medizinischen Behandlungsapparatur mit Hilfe von Markern und mit Hilfe eines Kamerasystems (Trackingsystem) detektiert wird, um eine bild unterstützte Chirurgie durchführen zu können.

Es lassen sich insbesondere hierbei geringere Gesamtsystemkosten erzielen, es können extrem kleine und leichte Marker verwendet werden, die Marker-Erfassungsprozeduren werden vereinfacht, die Genauigkeit wird erhöht und die Markererfassung wird robuster und vertrauenswürdiger.

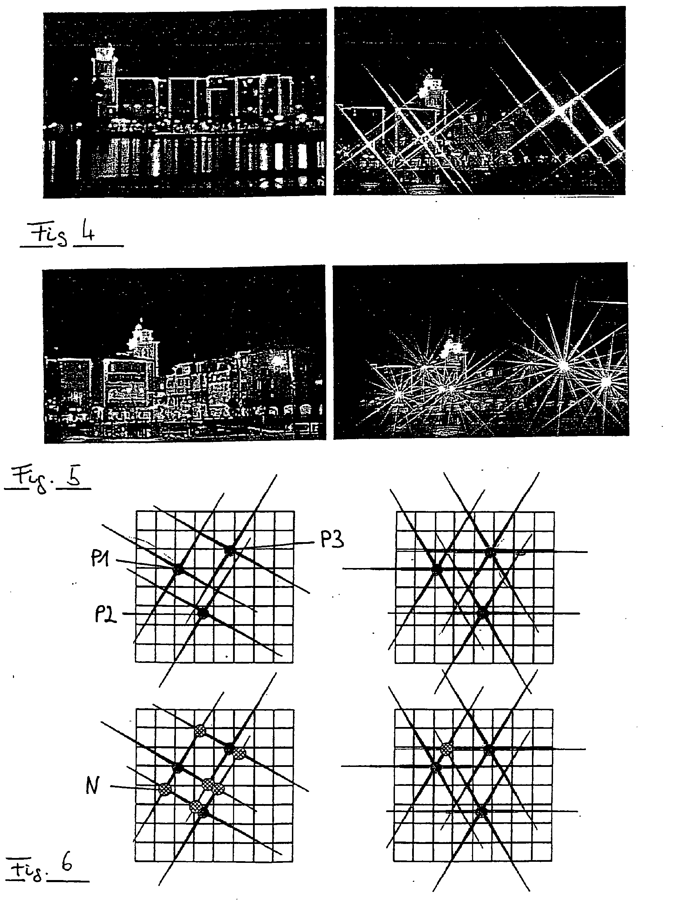

Lichtstreueffekte, nämlich im vorliegenden Beispiel Sterneffekte bzw. Kreuzeffekte, werden beispielsweise in den Figuren 4 und 5 aufgezeigt. Die beiden gegenübergestellten Bilder in den Figuren 4 und 5 zeigen grundsätzlich den Effekt eines Stern- bzw. Kreuzfilters in einem Bild mit mehreren hellen Bildpunkten (Spotlights). Wie ersichtlich wird, ergibt sich bei der Verwendung eines solchen Effektes an den Spotlights ein Kreuzmuster (Figur 4) oder ein Sternmuster (Figur 5), je nach Effekt-Ordnung. Der Kreuzungspunkt der senkrechten Strahlen (Figur 4) ist das Zentrum des Bildpunktes, also des Spotlights, nämlich der hellste Punkt. Wenn Unklarheiten über potentielle Bildpunktpositionen auftreten könnten, weil sich die Strahlen verschiedener Bildpunkte kreuzen und damit mehrere Kreuzungspunkte entstehen, kann dieses Problem basierend auf der Helligkeit bzw. dem Helligkeitsgradienten der Strahlen gelöst werden, oder durch die Einbeziehung von Informationen über die Strahlenform sowie durch die Verwendung von Stern- bzw. Kreuzfiltern höherer Ordnung, wie beispielsweise in Figur 5 gezeigt ist.

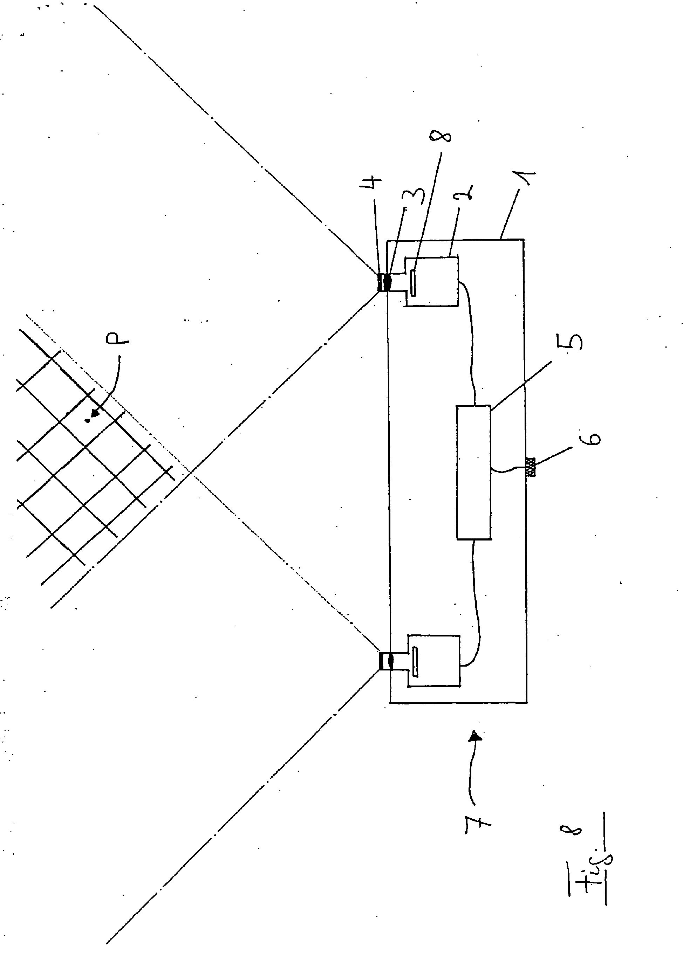

Erzielt werden kann der Stern- bzw. Kreuzeffekt auf verschiedene Weise, wie oben schon beschrieben wurde. Ein Beispiel ist die Verwendung eines Stern- oder Kreuzfilters, und wie dieser verwendet wird ist in Figur 8 zu sehen. Die Figur 8 zeigt die schematische Darstellung eines optischen, insbesondere eines medizinischen Trackingsystems 7. Dieses weist ein Gehäuse 1 auf, das mit zwei Kameras 2 bestückt ist. In der Figur 8 ist jedes Teil nur einmal mit einem Bezugszeichen versehen, auch wenn es, wie bei den Kameras, zweimal vorhanden ist.

Die Kameras 2 weisen einen gemeinsamen Sichtbereich auf, der kreuzschraffiert gezeichnet ist, und das Trackingsystem 7 kann, wenn es kalibriert ist, die dreidimensionale Position eines Bildpunktes in einem vorbestimmten Koordinatensystem ermitteln, wenn der Bildpunkt in dem gemeinsamen Sichtbereich liegt. Dazu geben die beiden Kameras 2 die zweidimensional ermittelte Position jeder Kamera an eine Bildverarbeitungseinheit 5 weiter, welche aus den beiden Informationen eine dreidimensionale Position ermittelt und diese über den Ausgang 6 ausgibt. An den Ausgang 6 kann beispielsweise ein medizinisches Navigationssystem angeschlossen werden, das dann eine bildgeführte Chirurgie ermöglicht, wenn mit dem Trackingsystem Marker an Patienten, Instrumenten oder medizinischen Apparaturen positionsmäßig ermittelt werden.

Bei der vorliegenden Ausführungsform weisen die Kameras 2 jeweils eine Linse 3 auf, die vor dem Bildsensor 8 liegt. Außen vor der Linse ist ein Stern- bzw. Kreuzeffekt-Filter aufgesetzt, der das Bezugszeichen 4 hat. Mit diesem Kamerasystem lässt sich nun also der Bildpunkt, beispielsweise der eingezeichnete Bildpunkt P positionsmäßig ermitteln, und im Weiteren wird zur Erläuterung wieder auf die Figur 7 Bezug genommen. Der Stern- bzw. Kreuzfilter kann zur Optimierung des Aufbaus auch mit geeigneten Oberflächenbearbeitungstechniken direkt auf das Linsensystem aufgebracht werden, oder kann sich in einer weiteren Form im bzw. hinter dem Linsensystem befinden.

Oben wurde schon beschrieben, welche Schwierigkeiten bei der herkömmlichen Bildpunkt-Positionsbestimmung auftreten, und zwar anhand der drei links untereinander angeordneten Bilder in Figur 7. Die drei rechts untereinander angeordneten Bilder zeigen nun den Effekt eines 4-fach-Sternfilters bei der Positionsbestimmung des gleichen Bildpunktes P, beispielsweise eines Markers. Dabei werden durch den Stern- bzw. Kreuzfilter Strahlen S1 und S2 erzeugt, welche sich zunächst als eine Anzahl von Pixelbelichtungen S1', S2' auf dem Sensor abbilden. Nunmehr ist eine viel größere Anzahl von Pixeln auf dem Sensor belichtet und aus den unterschiedlichen Helligkeiten lassen sich mit sehr viel höherer Genauigkeit wiederum die Strahlen S1" und S2" ermitteln, deren Kreuzungspunkt exakt die Position des Markers bzw. des Bildpunktes wiedergibt.

Im Einzelnen wird klar, dass die Interpolation der Position der Strahlen S1, S2, basierend auf der Helligkeit der zugeordneten Pixel eine sehr viel präzisere Schätzung des Schwerpunktes des Objektes bereitstellt, und zwar basierend auf der Tatsache, dass die Ausrichtung der Strahlen einerseits als gerade (und in diesem Fall senkrecht zueinander) bekannt ist, und zusätzlich die Richtung der Strahlen bekannt ist, nämlich definiert durch die Ausgestaltung des Stern- bzw. Kreuzfilters. Eine Objekt-Detektion (Segmentierung) kann deshalb dadurch vereinfacht werden, dass Kreuzungspunkte gerader Linien gesucht werden, bei denen der Winkel der Linien schon durch Gestaltungsvorgaben bekannt ist. Die Gesamt-Helligkeit des Markers wird über viele Pixel verteilt, und im vorliegenden Fall resultiert sie in einem geometrischen Muster, wobei der Kreuzungspunkt der geraden Linie das Zentrum des Markers ist. Das Bild des Markers könnte merklich kleiner sein als die Größe eines Pixels, da die Optik den Strahl über viele Pixel verteilt.

Vorteilhafterweise verlässt sich die Prozedur nicht länger auf Algorithmen, die einen Marker-Umriss erfassen müssen und aus diesen Informationen den Mittelpunkt finden. Die Kanten müssen nicht mehr exakt detektiert werden, da der Mittelpunkt des Markers basierend auf den tatsächlichen Helligkeitsgradienten und der Form der belichteten Pixel und zusätzlich aus der geometrischen Kreuzungspunkt-Information der Strahlen errechnet werden kann.

Basierend auf dieser Prozedur kann die Markergröße merklich reduziert werden, so dass ein Marker praktisch ein Punkt mit vernachlässigbarer Größe werden kann, solange er hell genug abstrahlt. Das Problem ungleichmäßig ausgeleuchteter, schmutziger oder abgedeckter Marker wird somit praktisch eliminiert.

Die Prozedur basiert auf den folgenden Annahmen:
- die Lichtquelle, welche das Stern- bzw. Kreuzmuster erzeugt, ist hell. In der Praxis kann die Lichtquelle Infrarot- oder UV-Licht emittieren oder reflektieren, so dass keine Irritation des Bedieners auftritt und Normallicht die Erfassung nicht stört;
- die Lichtquelle, welche das Stern- bzw. Kreuzmuster erzeugt, kann klein sein, so dass der Schwerpunkt definitiv der Helligkeits-Mittelpunkt ist;
- eine Abdeckung, Anfeuchtung, oder Verschmutzung des Markers bzw. Größentoleranzen sind vernachlässigbar, da der Marker in Bezug auf solche potentiellen Störungen sehr klein ist. Wenn ein Marker abgedeckt ist, ist es besser, er liefert keinen Bildpunkt statt einen falschen Bildpunkt. Der fehlende Bildpunkt kann z.B. durch einen weiteren (redundanten) Marker zur Verfügung gestellt werden;
- das Bild des Markers kann sehr klein sein, solange es hell genug ist, um ein Kreuzmuster aus mehreren Pixeln zu erzeugen.

Die oben genannten Annahmen sind sehr viel weniger kritisch als die oben angesprochenen Annahmen für die herkömmliche Bildpunkt-Positionserfassung (Schwerpunkt Berechnung), bei der die Form die Markers eine große Rolle spielt.

Oben wurde schon kurz eine Art Ausschlussverfahren angesprochen, mit dem verhindert wird, dass falsche Bildpunkte identifiziert werden. In Figur 6 ist ein solches Ausschlussverfahren nochmals dargestellt. Die beiden Darstellungen auf der linken Seite in Figur 6 zeigen oben die richtig identifizierten Bildpunkte P1, P2 und P3 an bestimmten Kreuzungspunkten mehrerer Lichtstrahlen. Im Bild darunter ist dargestellt, dass sich aber, wenn man lediglich die Kreuzungspunkte betrachtet, eigentlich mehrere Punkte ergeben würden, wobei einer als nicht echter Bildpunkt N bezeichnet ist. Die nicht echten Bildpunkte sind schraffiert gezeichnet.

Diese Situation könnte einerseits dadurch behoben werden, dass man Stern- bzw. Kreuzfilter mit höherer Ordnung verwendet, wie beispielsweise im Bild rechts oben in Figur 6. Mit dieser Maßnahme lässt sich dann sagen, dass nur dort, wo drei Strahlen sich kreuzen, tatsächlich ein Bildpunkt sein kann.

Auch in diesem Fall besteht noch die Möglichkeit, dass Unklarheiten bleiben. So ist im Bild rechts unten in Figur 6 gezeigt, dass der Punkt N ebenfalls der Kreuzungspunkt dreier Strahlen ist, obwohl er nicht tatsächlich einen Bildpunkt darstellt. In solchen Fällen hilft man sich, indem man die Helligkeit des Punktes n insgesamt zur Beurteilung heranzieht. Weil die Strahlen nach außen vom Zentrum weg an Helligkeit abnehmen wird der Punkt N nicht das gleiche Maß an Helligkeit aufweisen wie die tatsächlichen Bildpunkte, und er kann somit ausgeschlossen werden. Ein weiterer Hinweis für einen korrekten Marker ist die Tatsache, dass die Strahlen S1 uns S2 wie in Figur 7 rechts dargestellt am Kreuzungspunkt immer die gleiche Helligkeit haben. Dieses Kriterium ist für die Punkte N in Figur 6 nicht erfüllt. Die Ausschlussreihenfolge lautet also:
1. Kreuzungspunkt, 2. Kreuzungspunkt in der Ordnung des Stern- bzw. Kreuzfilters, 3. Kreuzungspunkt mit höchster Helligkeit und 4. Kreuzungspunkt mit gleicher Helligkeit der Strahlen.

Es lassen sich also in jedem Fall die richtigen Bildpunkte P1, P2 und P3 ermitteln.

## Patentansprüche

1. Positionsmarker-Trackingsystem mit einem optischen, insbesondere medizinischen Trackingsystem und mindestens einem lichtabstrahlenden Marker ( 24a-c ), der positionell von dem Trackingsystem erfassbar ist, wobei
der effektive abstrahlende Teil des oder der Marker ( 24a-c ) als Punktlichtquelle ausgebildet ist,
**dadurch gekennzeichnet, dass**
die Punktlichtquelle eine Größe von einem Bruchteil von 1 mm hat und
die Punktlichtquelle das Ende eines lichtleitenden Innenteils (33) einer Lichtleiterfaser (32; 33) umfasst , das so poliert oder geschliffen ist, dass es eine konvexe Streulinse bildet.

2. System nach Anspruch 1, **dadurch gekennzeichnet, dass** das oder die Punktlichtquelle eine Größe von mindestens 30µm, insbesondere mindestens 50µm und vorzugsweise mindestens 62,5µm hat bzw. haben.

3. System nach Anspruch 1 oder 2, **dadurch gekennzeichnet, dass** das oder die Punkilichtquelle so bearbeitet oder ausgebildet ist bzw. sind, dass sie Licht in räumlich gleichmäßiger Verteilung abstrahlen.

4. System nach einem der Ansprüche 1 bis 3, **dadurch gekennzeichnet, dass** es mehrere Punkilichtquelle (24a-c) umfasst, welche die Enden von Lichtleitern (23) sind, die aus einer gemeinsamen Lichtquelle gespeist werden, wobei die Lichtquelle eine aktiv abstrahlende Quelle oder eine Lichtsammeleinrichtung (22) sein kann, die Umgebungslicht sammelt.

## Claims

1. A positional marker tracking system comprising an optical, in particular medical tracking system and at least one light-emitting marker (24a-c) which can be positionally detected by the tracking system, wherein the actually emitting part of the marker or markers (24a-c) is formed as a point light source, **characterised in that** the point light source has a size of a fraction of 1 mm and the point light source comprises the end of a photoconductive inner part (33) of an optical fibre (32; 33) which is polished or burnished such that it forms a convex scattering lens.

2. The system according to claim 1, **characterised in that** the point light source(s) has/have a size of at least 30 um, in particular at least 50 µm and preferably at least 62.5 µm.

3. The system according to claim 1 or 2, **characterised in that** the point light source(s) is/are processed or formed such that it/they emit(s) light in a spatially uniform distribution.

4. The system according to any one of claims 1 to 3, **characterised in that** it comprises a number of point light sources (24a-c) which are the ends of optical fibres (23) fed from a common light source, wherein the light source can be an actively emitting source or a light collecting means (22) which collects ambient light.

## Revendications

1. Système de poursuite à marqueur de position comprenant un système de poursuite optique, en particulier médical, et au moins un marqueur (24a-c) émettant de la lumière, qui peut être détecté en position par le système de poursuite,
la partie émettrice efficace du ou des marqueur(s) (24 a-c) étant conçue comme source lumineuse ponctuelle,
**caractérisé en ce que**
la source lumineuse ponctuelle a une grandeur d'une fraction de 1 mm et
la source lumineuse ponctuelle comprend l'extrémité d'une partie intérieure (33) photoconductrice d'une fibre optique (32 ; 33), qui est polie ou poncée de telle sorte qu'elle forme une lentille divergente convexe.

2. Système selon la revendication 1, **caractérisée en ce que** la ou les source(s) lumineuse(s) ponctuelle(s) a ou ont une grandeur d'au moins 30 µm, en particulier au moins 50 µm et de préférence au moins 62,5 µm.

3. Système selon la revendication 1 ou 2, **caractérisé en ce que** la ou les source(s) lumineuse(s) ponctuelle(s) est ou sont traitée(s) ou conçue(s) de telle sorte qu'elle(s) émet(tent) de la lumière dans une répartition uniforme au plan spatial.

4. Système selon l'une des revendications 1 à 3, **caractérisé en ce qu'**il comprend plusieurs sources lumineuses ponctuelles (24a-c), lesquelles sont les extrémités de guides de lumière (23) qui sont alimentés à partir d'une source lumineuse commune, la source lumineuse pouvant être une source émettant de façon active ou un dispositif collecteur de lumière (22) qui collecte de la lumière ambiante.
